# NEW EUROPEAN PATENT SPECIFICATION

(11) **EP 0 841 946 B2**
(45) Date of publication and mention of the opposition decision: **15.10.2008**
(45) Mention of the grant of the patent: 02.10.2002
(21) Application number: 96926134.6
(22) Date of filing: 24.07.1996
(51) Int. Cl.: A61K 39/395

(54) **METHODS FOR TREATMENT OF ALLERGIC ASTHMA**
VERFAHREN ZUR BEHANDLUNG VON ALLERGISCHEM ASTHMA
PROCEDES DE TRAITEMENT DE L'ASTHME ALLERGIQUE

(30) Priority: 27.07.1995 US 508014
(43) Date of publication of application: 20.05.1998
(73) Proprietor: Genentech, Inc., South San Francisco CA 94080-4990 (US)
(72) Inventor: FICK, Robert, B., Jr., Portola Valley, CA 94028 (US); JARDIEU, Paula, M., San Francisco, CA 94109 (US); SCHOENHOFF, Monika, B., Redwood City, CA 94062 (US); SHIRE, Steven, J., Belmont, CA 94002 (US)
(74) Representative: Kremer, Simon Mark
(86) International application number: PCT/US1996/012275
(87) International publication number: WO 1997/004807

(56) References cited:
- EP-A- 0 317 295
- EP-A- 0 589 840
- EP-A- 0 648 499
- WO-A-89/06138
- WO-A-93/19197
- THE JOURNAL OF ALLERGY AND CLINICAL IMMUNOLOGY, vol. 95, no. 1 part 2, January 1995, ST. LOUIS, MO, USA, page 356 XP000611499 J. FROEHLICH ET AL.: "Multiple doses of a recombinant humanized monoclonal anti-IgE antibody are safely tolerated and decrease free serum IgE to undetectable levels."
- INTERNATIONAL ARCHIVES OF ALLERGY AND IMMUNOLOGY, vol. 107, no. 1-3, May 1995, BASEL, SWITZERLAND, pages 308-312, XP000611560 R. SHIELDS ET AL.: "Inhibition of allergic reactions with antibodies to IgE."
- THE JOURNAL OF ALLERGY AND CLINICAL IMMUNOLOGY, vol. 94, no. 5, November 1994, ST. LOUIS, MO, USA, pages 836-843, XP000611550 R. SABAN ET AL.: "Human FcERI-IgG and humanized anti-IgE monoclonal antibody MaE11 block passive sensitization of human and rhesus monkey lung."
- BIO/TECHNOLOGY, vol. 8, February 1990, NEW YORK, NY, USA, pages 122-126, XP002019200 T. CHANG ET AL.: "Monoclonal antibodies specific for human IgE-producing B cells: a potential therapeutic for IgE-mediated allergic diseases."
- ANNALS OF THE NEW YORK ACADEMY OF SCIENCES, vol. 685, 23 June 1993, NEW YORK, NY, USA, pages 549-560, XP000611552 S. NAKAJIMA ET AL.: "Effect of saiboku-to (TJ-96) on bronchial asthma."
- THE JOURNAL OF ALLERGY AND CLINICAL IMMUNOLOGY, vol. 97, no. 1 part 3, January 1996, ST. LOUIS, MO, USA, page 315 XP000611551 D. COCKCROFT ET AL.: "rhuMAb-E25 (E25), humanized murine monoclonal anti-IgE, inhibits the allergen-induced early asthmatic response (EAR)."

## Description

### FIELD OF THE INVENTION

This invention relates to methods of treatment of allergic asthma with IgE antagonists, including anti-IgE antibodies.

### BACKGROUND OF THE INVENTION

Asthma is characterized by three components: airways inflammation: airways obstruction, which is reversible; and increased sensitivity, referred to as hyperreactivity. Obstruction to airflow is measured by a decrement in forced expired volume in one second (FEV₁) which is obtained by comparison to baseline spirometry. Hyperreactivity of the airways is recognized by decreases in FEV₁ in response to very low levels of histamine or methacholine. Hyperreactivity may be exacerbated by exposure of the airways to allergen.

People with allergic asthma who inhale an aerosolized allergen to which they are sensitive develop immediate "early asthmatic response" (EAR), and often delayed "late asthmatic response" (LAR), as measured by FEV₁ (Cockcroft et al. Clin. Allergy 7:503-13 (1977); Hargreave et al. J. Allergy Clin. Immunol. 83:525-7 (1989)). The EAR results from mast cell degranulation provoked by cross linking of an antigen with mast cell-bound IgE. Preformed mediators (such as histamine and tryptase) and newly formed lipid mediators (such as prostaglandins and leukotrienes) released from mast cells cause bronchoconstriction, mucus hypersecretion, and changes in vascular permeability (Fick et al. J. Appl. Phys. 63:1147-55 (1987)). EAR recovery occurs within 30-60 minutes. The LAR is associated with more pronounced airway inflammation characterized by eosinophilic and neutrophilic infiltration of the mucosa, more prolonged changes in bronchovascular permeability, and increased bronchial reactivity to non-specific stimuli (Hargreave et al., supra: Fick et al. supra: Diaz et al. Am. Rev. Respir. Dis. 139:1383-9 (1989); Fahy et al. J. Allergy Clin. Immunol. 93:1031-9 (1994). The pathophysiologic relationship between LAR and EAR remains unclear, but the LAR airway changes may be caused by mast cell mediators (including cytokines) released during EAR. The activities of these mast cell mediators include chemoattraction of inflammatory cells to the airway mucosa and the induction of more prolonged changes in vascular permeability in the airway mucosa (Fick et al. Am. Rev. Respir. Dis. 135:1204-9 (1987)).

IgE is thought to be the central effector antibody in the EAR to inhaled allergen in people with allergic asthma, although its role in the LAR is unclear. Chronic allergic asthma may result from continual degranulation of airway mast cells in response to continued exposure to perennial allergens (i.e., the house dust mite, dog dander, and cat hair). This hypothesis is supported by studies which demonstrate a reduction in asthma symptoms and in bronchial hyperactivity in subjects who reduce their environmental exposure to aeroallergens (Plans-Mills et al. Lancet ii:675-8 (1982); Murray et al. Pediatrics 71:418-22 (1983)).

Airway hyperreactivity reactions can be induced in the laboratory by exposing subjects with allergic asthma to nebulized solutions of allergen extract, the concentration of which can be determined by airway hyperreactivity to methacholine and skin test reactivity to the same allergen. This procedure is known as experimental aerosolized allergen challenge or bronchial provocation. Bronchial provocation is a useful and relevant model for the study of anti-asthma medications (Cockcroft et al. J. Allergy Clin. Immunol. 79:734-40 (1987); Cresciolli et al. Ann. Allergy 66:245-51 (1991); Ward et al. Am. Rev. Respir. Dis. 147:518-23 (1993)). For example, it is known that beta agonists inhibit the EAR but not the LAR to allergen, and that a single dose of inhaled steroid inhibits the LAR but not the EAR (Cockcroft et al.. J. Allergy Clin. Immunol. 79:734-40 (1987)). Theophylline and disodium cromoglycate attenuate both the EAR and LAR responses to allergen (Cresciolli et al., supra; Ward et al. supra). Most drugs with proven efficacy in asthma management have been shown to attenuate airway responses to inhaled antigens administered in bronchial provocation. If airway mast cell-bound IgE is central to the airway response to inhaled allergen, then decreasing or eliminating circulating and mast cell bound IgE may result in significant attenuation of the EAR and possibly also the LAR to inhaled aeroallergens.

The concept of using anti-IgE antibodies as a treatment for allergy has been widely disclosed in the scientific literature. A few representative examples are as follows. Baniyash and Eshhar (European Journal of Immunology 14:799-807 (1984)) demonstrated that an anti-IgE monoclonal antibody could specifically block passive cutaneous anaphylaxis reaction when injected intradermally before challenging with the antigen; U.S. 4,714,759 discloses a product and process for treating allergy, using an antibody specific for IgE; and Rup and Kahn (International Archives Allergy and Applied Immunology, 89:387-393 (1989) discuss the prevention of the development of allergic responses with monoclonal antibodies which block mast cell-IgE sensitization.

Anti-IgE antibodies which block the binding of IgE to its receptor on basophils and which fail to bind to IgE bound to the receptor, thereby avoiding histamine release are disclosed, for example, by Rup and Kahn (supra), by Baniyash et al. (Molecular Immunology 25:705-711, 1988), and by Hook et al. (Federation of American Societies for Experimental Biology, 71st Annual Meeting, Abstract #6008, 1987).

Antagonists of IgE in the form of receptors, anti-IgE antibodies, binding factors, or fragments thereof have been disclosed in the art. For example, U.S. 4,962,035 discloses DNA encoding the alpha-subunit of the mast cell IgE receptor or an IgE binding fragment thereof. Hook et al. (Federation Proceedings Vol. 40, No. 3, Abstract #4177) disclose monoclonal antibodies, of which one type is anti-idiotypic, a second type binds to common IgE determinants, and a third type is directed towards determinants hidden when IgE is on the basophil surface.

U.S. 4,940,782 discloses monoclonal antibodies which react with free IgE and thereby inhibit IgE binding to mast cells, and react with IgE when it is bound to the B-cell FcE receptor, but do not bind with IgE when it is bound to the mast cell FcE receptor, nor block the binding of IgE to the B-cell receptor.

U.S. 4,946,788 discloses a purified IgE binding factor and fragments thereof, and monoclonal antibodies which react with IgE binding factor and lymphocyte cellular receptors for IgE, and derivatives thereof.

U.S. 5,091,313 discloses antigenic epitopes associated with the extracellular segment of the domain which anchors immunoglobulins to the B cell membrane. The epitopes recognized are present on IgE-bearing B cells but not basophils or in the secreted, soluble form of IgE. U.S. 5,252,467 discloses a method for producing antibodies specific for such antigenic epitopes. U.S. 5,231,026 discloses DNA encoding murine-human antibodies specific for such antigenic epitopes.

U.S. 4,714,759 discloses an immunotoxin in the form of an antibody or an antibody fragment coupled to a toxin to treat allergy.

Presta et al. (J. Immunol. 151:2623-2632 (1993)) disclose a humanized anti-IgE antibody that prevents the binding of free IgE to FceRI but does not bind to Fc∈RI-bound IgE. Copending WO93/04173 discloses polypeptides which bind differentially to the high- and low-affinity IgE receptors.

U.S. 5,428,133 discloses anti-IgE antibodies as a therapy for allergy, especially antibodies which bind to IgE on B cells, but not IgE on basophils. This publication mentions the possibility of treating asthma with such antibodies. U.S. 5,422,258 discloses a method for making such antibodies.

Tepper et al. ("The Role of Mast cells and IgE in Marine Asthma", presented at "Asthma Theory to Treatment", July 15-17, 1995) disclose that neither mast cells nor IgE greatly influence the anaphylaxis, airway hyperreactivity, or airway inflammation in a murine model of asthma.

EP-A-0648499 describes a specific peptide which is capable of binding human IgE, inhibits the 'production of IgE and can block allergic reactions such as bronchial asthma and atopy dermatitis.

EP-A-0317295 discloses that 4H-quinolizin-4-one compounds inhibit IgE formation and may be used for the treatment of bronchial asthma, atopy dermatitis and hypersensitiveness.

Shields et al, Int. Arch. Allergy Immunol (1995); 107:308-312 describes the use of anti-IgE humanized mAb E25 in the treatment of, for example, allergic asthma.

Nakajima et al, Annals New York Academy of Sciences (1993) 685:549-560 describes a specific plant extract with a steroid-like effect, which can suppress the induction of IgE-Fc∈R/CD23 in human lymphocytes by mite allergen, and reduces IgE production by inhibiting the production of IL-4 from T_{H}2 cells. In a guinea-pig model of asthma, late asthmatic response, as measured by leukocytes in bronchoalveolar lavage fluid, is reduced.

WO-A-89/06138 describes a mAb which specifically binds IgE-bearing B cells, allowing the targeting and elimination of these B cells. It is stated that the mAb may be used against IgE-mediated allergies such as extrinsic bronchial asthma.

Froehlich *et al*, J. Allergy Clin. Immunol. (1995) 95 (1, part 2) abstract 863 discloses that multiple doses of anti-IgE recombinant humanised mAb E25 may be safely tolerated, does not induce anaphylaxis, and may decrease free serum IgE levels to undetectable levels.

### SUMMARY OF THE INVENTION

Described herein is a method of treatment of allergic asthma in a patient comprising administering to the patient a maintenance dose of an IgE antagonist and, optionally, a loading dose of the IgE antagonist

Also described is a method for treating allergic asthma in a patient comprising administering to the patient a dose of IgE antagonist averaging about 0.001 to 0.01 mg/kg/week IgE antagonist for every IU/ml baseline IgE in the patient's serum.

According to the invention there is provided the use of an IgE antagonist in the preparation of a pharmaceutical composition for the reduction of the late asthmatic response of allergic asthma comprising a maintenance dose of an IgE antagonist, and optionally, a loading dose of the IgE antagonist, wherein the IgE antagonist acts by blocking the binding of IgE to its receptors on B cells, mast cells, or basophils by blocking the binding site on the IgE molecule; by binding soluble IgE; or by binding to IgE on B cells. In this and all other aspects and embodiments of the claimed invention the IgE antagonist is an anti-IgE antibody.

Preferably the maintenance dose may average about 0.001 to 0.01 mg/kg/week IgE antagonist for every IU/ml baseline IgE in the patient's serum.

Also described is a method of reducing the early asthmatic response in a patient comprising administering to the patient a maintenance dose of an IgE antagonist and, optionally, a loading dose of the IgE antagonist

In this method the dose of IgE antagonist may average about 0.001 to 0.01 mg/kg/week IgE antagonist for every IU/ml baseline IgE in the patient's serum.

Further described is a method of reducing bronchial hyperreactivity in a patient comprising administering to the patient a maintenance dose of an IgE antagonist and, optionally a loading dose of the IgE antagonist.

In this method the dose of IgE antagonist may average about 0.001 to 0.01 mg/kg/week IgE antagonist for every IU/ml baseline IgE in the patient's serum.

Also described is a method of reducing skin reactivity in a patient comprising administering to the patient a maintenance dose of an IgE antagonist and, optionally, a loading dose of the IgE antagonist.

In this method the dose of IgE antagonist may average about 0.001 to 0.01 mg/kg/week IgE antagonist for every IU/ml baseline IgE in the patient's serum.

Further described is a method of reducing lung inflammation in a patient comprising administering to the patient a maintenance dose of an IgE antagonist and, optionally, a loading dose of the IgE antagonist.

In this method the dose of IgE antagonist may be about 0.001 to 0.01 mg/kg/week IgE antagonist for every IU/ml baseline IgE in the patient's serum.

### BRIEF DESCRIPTION OF THE FIGURES

Figure 1 is a graph depicting the percentage change in FEV₁ from baseline in an allergen bronchial challenge in anti-IgE antibody treated patients and patients receiving placebo in the U.S. dosing protocol.

Figures 2 (U.S.) and 3 (Canada) depict the results from methacholine bronchial challenge in anti-IgE antibody treated patients and patients receiving placebo.

Figures 4 (U.S.) and 5 (Canada) depict the change from baseline in total symptoms scores in anti-IgE antibody treated patients and patients receiving placebo.

Figures 6 (U.S.) and 7 (Canada) depict endpoint titration skin testing for allergens in patients receiving placebo or anti-IgE antibody.

### DETAILED DESCRIPTION OF THE INVENTION

### A. DEFINITIONS

The term "asthma" as used herein refers to a lung disease characterized by airway obstruction that is reversible (although not entirely in some patients) either spontaneously or with treatment, airway inflammation, and increased airway responsiveness to a variety of stimuli. "Allergic asthma" as used herein refers to an asthmatic response to inhalation of an antigen to which the patient is sensitive.

The term "early asthmatic response" (EAR) as used herein refers to an asthmatic response to an antigen within about two hours of exposure. The term "late asthmatic response" (LAR) as used herein refers to an asthmatic response to an antigen within about two to eight hours after exposure.

The term "IgE antagonist" as used herein refers to a substance which inhibits the biological activity of IgE. Such antagonists are anti-IgE antibodies. Antibody antagonists may be of the IgA, IgD, IgG, IgE, or IgM class.

In general, in some embodiments of the invention, IgE antagonists act by blocking the binding of IgE to its receptors on B cells, mast cells, or basophils, by blocking the binding site on the IgE molecule. Additionally, in some embodiments of the invention, IgE antagonists act by binding soluble IgE and thereby removing it from circulation. The IgE antagonists of the invention can also act by binding to IgE on B cells, thereby eliminating clonal populations of B cells. The IgE antagonists of the instant invention can also act by inhibiting IgE production. Preferably, the IgE antagonists of the instant invention do not result in histamine release from mast cells or basophils.

The term "therapeutic amount" as used herein denotes an amount that prevents or ameliorates symptoms of a disorder or responsive pathologic physiological condition.

"Polypeptide" as used herein refers generally to peptides and proteins having at least about two amino acids.

The term "free IgE" as used herein refers to IgE not complexed to a binding partner, such an anti-IgE antibody. The term "total IgE" as used herein refers to the measurement of free IgE and IgE complexed to a binding partner, such as an anti-IgE antibody. The term "baseline IgE" as used herein refers to the level of free IgE in a patient's serum before treatment with an IgE antagonist.

The term "polyol" as used herein denotes a hydrocarbon including at least two hydroxyls bonded to carbon atoms, such as polyethers (e.g. polyethylene glycol), trehalose, and sugar alcohols (such as mannitol).

The term "polyether" as used herein denotes a hydrocarbon containing at least three ether bonds. Polyethers can include other functional groups. Polyethers useful for practicing the invention include polyethylene glycol (PEG).

### B. GENERAL METHODS

Polyclonal antibodies to IgE generally are raised in animals by multiple subcutaneous (sc) or intraperitoneal (ip) injections of IgE and an adjuvant. It can be useful to conjugate IgE or a fragment containing the target amino acid sequence from the Fc region of IgE to a protein that is immunogenic in the species to be immunized, e.g., keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOCl₂, or R¹N = C = NR, where R and R¹ are different alkyl groups.

Animals ordinarily are immunized against the cells or immunogenic conjugates or derivatives by combining 1 mg or 1 µg of IgE with Freund's complete adjuvant and injecting the solution intradermally at multiple sites. One month later the animals are boosted with 1/5 to 1/10 the original amount of conjugate in Freund's incomplete adjuvant by subcutaneous injection at multiple sites. Seven to 14 days later, animals are bled and the serum is assayed for anti-IgE titer. Animals are boosted until the titer plateaus. Preferably, the animal is boosted with a conjugate of the same IgE, but conjugated to a different protein and/or through a different cross-linking agent Conjugates also can be made in recombinant cell culture as protein fusions. Also, aggregating agents such as alum can be used to enhance the immune response.

Monoclonal antibodies are prepared by recovering spleen cells from immunized animals and immortalizing the cells in conventional fashion, e.g. by fusion with myeloma cells or by Epstein-Barr (EB)-virus transformation and screening for clones expressing the desired antibody. The hybridoma technique described originally by Koehler and Milstein, Eur. J. Immunol., 6: 511 (1976) and also described by Hammerling et al., in: Monoclonal Antibodies and T-Cell Hybridomas. Elsevier, N.Y., pp. 563-681 (1981) has been widely applied to produce hybrid cell lines that secrete high levels of monoclonal antibodies against many specific antigens.

The hybrid cell lines can be maintained *in vitro* in cell culture media. The cell lines producing the antibodies can be selected and/or maintained in a composition comprising the continuous cell line in hypoxanthine-aminopterin thymidine (HAT) medium. In fact, once the hybridoma cell line is established, it can be maintained on a variety of nutritionally adequate media. Moreover, the hybrid cell lines can be stored and preserved in any number of conventional ways, including freezing and storage under liquid nitrogen. Frozen cell lines can be revived and cultured indefinitely with resumed synthesis and secretion of monoclonal antibody.

The secreted antibody is recovered from tissue culture supernatant by conventional methods such as precipitation, ion-exchange chromatography, affinity chromatography, or the like. The antibodies described herein are also recovered from hybridoma cell cultures by conventional methods for purification of IgG or IgM. as the case may be, that heretofore have been used to purify these immunoglobulins from pooled plasma, e.g., ethanol or polyethylene glycol precipitation procedures. The purified antibodies are sterile-filtered.

While routinely mouse monoclonal antibodies are used, the invention is not so limited: in fact, human antibodies can be used. Such antibodies can be obtained, for example, by using human hybridomas (Cote et al., Monoclonal Antibodies and Cancer Therapy, Alan R. Liss, p. 77 (1985)). In fact. according to the invention. techniques developed for the production of chimeric antibodies (Cabilly et al., U.S. 4,816,567, Morrison et al., Proc. Natl. Acad. Sci. 81:6851 (1984); Boulianne et al., Nature 312:643-646 (1984): Neuberger et al., Nature. 312: 604 (1984); Neuberger et al., Nature 314: 268-270 (1985); Takeda et al., Nature 314: 452 (1985); EP 184,187; EP 171,496; EP 173,494; PCT WO 86/01533: Shaw et al., J. Nat. Canc. Inst. 80:1553-1559 (1988); Morrison, Science 229: 1202-1207 (1985); Oi et al., BioTechniques, 4: 214 (1986)) by coupling an animal antigen-binding variable domain to a human constant domain can be used; such antibodies are within the scope of this invention. The term "chimeric" antibody is used herein to describe a polypeptide comprising at least the antigen binding portion of an antibody molecule linked to at least part of another protein (typically an immunoglobulin constant domain).

In one embodiment, such chimeric antibodies contain about one third rodent (or other non-human species) sequence and thus are capable of eliciting a significant anti-globulin response in humans. For example, in the case of the murine anti-CD3 antibody OKT3, much of the resulting anti-globulin response is directed against the variable region rather than the constant region (Jaffers et al., Transplantation 41: 572-578 (1986)).

Humanized antibodies are used to reduce or eliminate any anti-globulin immune response in humans. In practice, humanized antibodies are typically human antibodies in which some amino acid residues from the complementarity determining regions (CDRs), the hypervariable regions in the variable domains which are directly involved with formation of the antigen-binding site, and possibly some amino acids from the framework regions (FRs), the regions of sequence that are somewhat conserved within the variable domains, are substituted by residues from analogous sites in rodent antibodies. The construction of humanized antibodies is described in Riechmann et al., Nature 332: 323-327 (1988), Queen et al., Proc. Natl. Acad. Sci. USA 86: 10029-10033 (1989), Co et al., Proc. Natl. Acad. Sci. USA 88: 2869-2873 (1991), Gorman et al., Proc. Natl. Acad. Sci. 88: 4181-4185 (1991), Daugherty et al, Nucleic Acids Res. 19: 2471-2476 (1991), Brown et al., Proc. Natl. Acad. Sci. USA 88:2663-2667 (1991), Junghans et al., Cancer Res. 50:1495-1502 (1990), Fendly et al., Cancer Res. 50: 1550-1558 (1990) and in PCT applications WO 89/06692 and WO 92/22653.

In some cases, substituting CDRs from rodent antibodies for the human CDRs in human frameworks is sufficient to transfer high antigen binding affinity (Jones et al., Nature 321: 522-525 (1986); Verhoeyen et al., Science 239: 1534-1536 (1988)) whereas in other cases it is necessary to additionally replace one (Riechmann et al., supra) or several (Queen et al., supra) FR residues. See also Co et al., supra.

The invention also encompasses the use of human antibodies produced in transgenic animals. In this system. DNA encoding the antibody of interest is isolated and stably incorporated into the germ line of an animal host. The antibody is produced by the animal and harvested from the animal's blood or other body fluid. Alternatively, a cell line that expresses the desired antibody can be isolated from the animal host and used to produce the antibody in vitro, and the antibody can be harvested from the cell culture by standard methods.

The amount of IgE antagonist delivered to the patient to be used in therapy will be formulated and dosages established in a fashion consistent with good medical practice taking into account the disorder to be treated, the condition of the individual patient, the site of delivery, the method of administration and other factors known to practitioners. Similarly, the dose of the IgE antagonist administered will be dependent upon the properties of the IgE antagonist employed, e.g. its binding activity and in vivo plasma half-life, the concentration of the IgE antagonist in the formulation, the administration route, the site and rate of dosage, the clinical tolerance of the patient involved, the pathological condition afflicting the patient and the like, as is well within the skill of the physician.

Typically IgE antagonists are administered by intramuscular, intravenous, intrabronchial, intraperitoneaL subcutaneous or other suitable routes. The antagonists can be administered before and/or after the onset of symptoms. In general, a "loading" dose of an IgE antagonist is useful to obtain a rapid and sustained decrease in free IgE. A loading dose is typically a first dose of IgE antagonist that is greater than a subsequent or "maintenance" dose of IgE antagonist However, patients can be loaded in other ways. For example, patients can be loaded by administering a dose of antagonist that is greater than or equal to the same mg/kg amount as the maintenance dose. but increasing the frequency of administration in a "loading regimen". Thus, for example, if the maintenance dose is 1 mg/kg biweekly, the patient can be loaded by administering 1 mg/kg weekly for two or more weeks in a row, then administering the maintenance dose of 1 mg/kg biweekly. Furthermore, patients can be loaded during a course of treatment with a maintenance dose of IgE antagonist by administering larger or more frequent doses than the maintenance dose. The term "loading dose" is intended as used herein to include such single loading doses, multiple loading doses, loading regimens, and combinations thereof.

A sustained decrease in free IgE can be obtained by administration of a maintenance dose of the antagonist. Maintenance doses are delivered with a frequency of about every day to about every 90 days, more preferably weekly to biweekly, depending on the severity of the patient's symptoms, the concentration and in vivo properties of antagonist delivered, and the formulation of the antagonist. For example, slow release formulations can allow less frequent administration. Maintenance doses can be adjusted upwards or downwards over time, depending on the response of the patient.

Thus, for example. in one embodiment of the invention, the dose of IgE antagonist is sufficient to reduce free IgE in the patient's serum to less than about 40 ng/ml.

In a further dosing strategy, about 0.05 to 10 mg/kg, more preferably about 0.1 to 1 mg/kg, most preferably about 0.5 mg/kg IgE antagonist can be administered on a weekly basis to a patient having about 40-200 IU/ml baseline IgE. In another dosing strategy for individuals with higher baseline IgE, patients are preferably "loaded" with about 1 to about 10 mg/kg, more preferably about 1 to about 5 mg/kg, most preferably about 2 mg/kg, IgE antagonist, followed by weekly or biweekly administration of about 0.1 to about 10 mg/kg, most preferably about 1 mg/kg.

In a further dosing strategy, a maintenance dose of IgE antagonist averaging about 0.0005 to 0.05 mg/kg/week for every IU/ml baseline IgE, more preferably 0.001 to about 0.01 mg/kg/week for every IU/ml baseline IgE is used. This maintenance regimen can follow an initial loading dose of about I to 10 mg/kg, more preferably about 1 to 5 mg/kg IgE antagonist.

In a further embodiment of the invention, sufficient IgE antagonist is provided through the maintenance dose, and, optionally, the loading dose, to achieve about a 1 to 20 fold, preferably about 3 to 5 fold, most preferably about a 5 fold greater serum concentration than total serum IgE concentration in the patient.

IgE levels are typically assayed by standard ELISA techniques well known in the art. Total serum IgE can be measured by commercially available assays, such as Abbott Laboratories' Total IgE assay. Free IgE, e.g., IgE not bound to antibody can be measured by a capture type assay in which, for example, IgE receptor is bound to a solid support. IgE complexed to an anti-IgE antibody which binds at or near the site on IgE which binds to the receptor will be blocked from binding the receptor, and thus only free or unbound IgE can react with the receptor bound to the solid support in this assay. An anti-IgE antibody which recognizes IgE even when the IgE is bound to its receptor can be used to detect the IgE captured by the receptor on the solid support. This anti-IgE antibody can be labeled with any of a variety of reporter systems, such as alkaline phosphatase, etc.

It is envisioned that injections (intravenous, intramuscular or subcutaneous) will be the primary route for therapeutic administration of the IgE antagonist of this invention, although delivery through catheter or other surgical tubing is also used. Alternative routes include suspensions, tablets, capsules and the like for oral administration, commercially available nebulizers for liquid formulations, and inhalation of lyophilized or aerosolized microcapsules, and suppositories for rectal or vaginal administration. Liquid formulations can be utilized after reconstitution from powder formulations.

Additional pharmaceutical methods may be employed to control the duration of action of the antagonists of this invention. The antagonists also may be entrapped in microcapsules prepared. for example, by coacervation techniques or by interfacial polymerization (for example, hydroxymethylcellulose or gelatin-microcapsules and poly-(methylmethacylate) microcapsules, respectively), in colloidal drug delivery systems (for example, liposomes, albumin microspheres, microemulsions, nano-particles and nanocapsules), or in macroemulsions. Such techniques are disclosed in Remington's Pharmaceutical Sciences, 16th edition, Osol, A., ed., 1980).

In general, the formulations of the subject invention can contain other components in amounts not detracting from the preparation of stable forms and in amounts suitable for effective, safe pharmaceutical administration. For example, other pharmaceutically acceptable excipients well known to those skilled in the art can form a part of the subject compositions. These include, for example, salts, various bulking agents, additional buffering agents, chelating agents, antioxidants, cosolvents and the like; specific examples of these include tris-(hydroxymethyl)aminomethane salts ("Tris buffer"), and disodium edetate.

In one embodiment of the invention. IgE antagonist formulations comprise a buffer, a salt, optionally, a polyol, and optionally, a preservative.

One exemplary formulation of the invention is a liquid formulation of about 1-100 mg/ml IgE antagonist in 10 mM acetate buffer, pH 5.0-6.5, 100-200 mM sodium chloride, and about 0.01% polysorbate 20, more preferably about 5 mg/ml IgE antagonist in 10 mM acetate buffer, pH 52,142 mM sodium chloride, and 0.01% polysorbate 20. In other embodiments of the invention, the formulation may be freeze-dried and reconstituted for administration. For example, anti-IgE antibody can be formulated at about 25 mg/ml in 5 mM histidine, pH 6.0, and 88 mM sucrose, freeze-dried, and reconstituted in water to 100 mg/ml antibody for administration. Mixed sugars can also be used, such as a combination of sucrose and mannitol, etc.

In general, unless otherwise specified, the abbreviations used for the designation of amino acids and the protective groups used therefor are based on recommendations of the IUPAC-IUB Commission of Biochemical Nomenclature (Biochemistry, 11:1726-1732 (1972). The nomenclature used to define compounds of the invention is that specified by the IUPAC, published in European Journal of Biochemistry 138:9-37 (1984).

Therapy of allergic asthma can be combined with other known therapies for allergy and/or asthma, including anti-histamines, theophylline, salbutamol, beclomethasone dipropionate, sodium cromoglycate, steroids, anti-inflammatory agents, etc.

Further details of the invention can be found in the following examples, which farther define the scope of the invention. All references cited herein are expressly incorporated by reference in their entireties.

### EXPERIMENTAL RESULTS

The objects of this randomized, double blind, placebo-controlled, multi-center Phase II clinical study were to determine if an IgE antagonist in the form of an anti-IgE antibody inhibits the EAR and/or the LAR to an inhaled aeroallergen in asthmatic patients. Additional parameters examined included inhibition of the increase in bronchial reactivity, inhibition of the increase in biologic markers of inflammation, and decrease in asthma symptoms in response to treatment with an anti-IgE antibody. The anti-IgE antibody E25 used in this study was the humanized version of monoclonal antibody MaEl I described in Presta et al., supra.

Two dosing protocols were used (denoted U.S. and Canada). In both protocols, the subjects underwent an initial (control) allergen diluent provocation challenge and then two allergen bronchial challenges separated by approximately eight weeks of study drug. 10 U.S. and 9 Canada subjects received anti-IgE antibody E25 and 9 U.S. and 9 Canada subjects received placebo therapy. The allergens used for the study were house dust mite, cat hair, or grass. The allergen used for each individual patient was the one that elicited the most positive response in that patient's allergen skin prick challenge test. On Day 0, the day after the first (baseline) allergen bronchial challenge, U.S. patients received 0.5 mg/kg E25 or placebo equivalent intravenously. U.S. patients subsequently received 0.5 mg/kg E25 or placebo equivalent intravenously weekly. Canada subjects received 2.0 mg/kg E25 or placebo equivalent on Day 0, 1.0 mg/kg E25 or placebo equivalent on days 7 and 14, and 1.0 mg/kg biweekly thereafter. In both the Canada and U.S. studies, one patient receiving E25 withdrew because of an asthma attack.

The airway effects of inhaled antigen solution was evaluated in three ways in the U.S. protocol and two ways in the Canada protocol. In the U.S. protocol, first, allergen-induced reductions in airflow dunng the EAR and LAR were recorded by measuring changes from baseline in FEV₁. Second, LAR changes in bronchial reactivity to methacholine were measured. Third, changes in inflammatory markers (eosinophil and neutrophil percent and number, eosinophil cationic protein, myeloperoxidase (MPO) and tryptase) in induced sputum were measured during the LAR.

In the Canada protocol, changes in the amount of aeroallergen required to provoke a greater than or equal to 15% decrease in FEV₁(PD₁₅) were quantitated. Second, changes in the methacholine concentration required to induce a greater than or equal to 20% fall in FEV₁(PD_{20 Mch}) were assessed a day prior to the first and last aeroallergen bronchoprovocation and on Day 42.

In the U.S., the primary efficacy variable was the change in FEV₁ measured within one hour of allergen challenge (EAR) between Day-1 and Day 63. The baseline was defined as the observed difference in the percent change from prechallenge levels in FEV₁ response between the Day-6 allergen diluent challenge and the Day-1 allergen challenge. Follow-up was defined as the difference between the Day 56 allergen diluent challenge and the Day 63 allergen diluent challenge. In each case, two variables were derived: maximal observed decrease and area under the curve (AUC) as approximated by the trapezoidal rule. Treatment efficacy was based on the between treatment comparison of the baseline and follow-up of AUC and maximal decrease. Between group differences for the change between Day-1 and Day 63 were assessed by the Wilcoxon Rank sum test.

The LAR was measured in a similar fashion as the primary efficacy variable of change in the FEV_{1 (AUC} and maximal decrease)

The initial dosing of allergen for inhalation was four doubling doses below that calculated from the prediction formula: y = 0.69x +0.11, where y = log₁₀ allergen PD₂₀FEV₁ (the dose of allergen that causes a 20% fall in FEV₁) and x = log₁₀ methacholine PD₁₀ (the dose of methacholine that causes a 10% fall in FEV₁) X skin allergen sensitivity (skin sensitivity to allergen is defined as the smallest allergen dilution that gives a wheal 2 mm in diameter). When the dose caused a fall in FEV₁ of 20% or more than no further allergen was delivered. When the dose caused a fall in FEV₁ of less than 10%, then the challenge advanced to the next doubling step, etc. The FEV₁ was measured at 20,30,45,60,90, and 120 minutes and at hourly intervals up to 7 hours after inhalation.

Dosing of allergen for the second bronchial challenge commenced at an allergen concentration of four doubling doses more dilute than the dose which caused a 20% fall in FEV₁ during the first challenge. The dosing then proceeded in two-fold more concentrated steps until the FEV₁ fell by 20% or until allergen was delivered at a concentration one doubling dose higher than that delivered on Day-1.

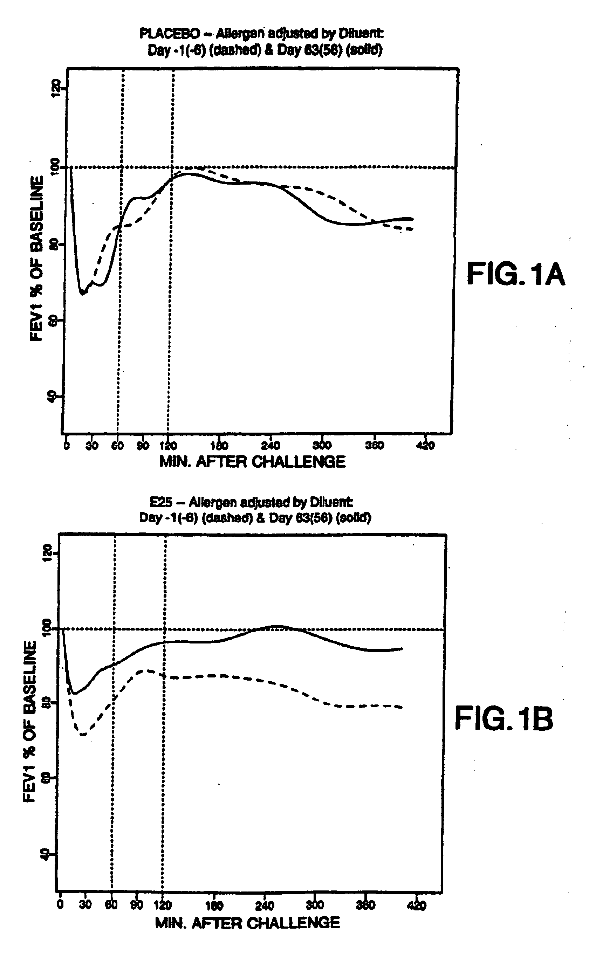

The results for FEV₁ percent change from baseline in allergen bronchial challenge in the U.S. dosing protocol are shown in tabular form in Tables I and II and in graphic form in Figure 1. The numerical values were adjusted by the diluent challenge. One patient withdrew from the study, reducing the total number enrolled to 19.

These data indicate that this treatment protocol with an anti-IgE antibody effectively reduced the EAR by 43% and the LAR by 82%.

**Table I.**

| **EAR FEV₁ Percent Change from Baseline in Allergen Challenge** | | |
|---|---|---|
| | Placebo | E25 |
| Total Number of Patients | 9 | 9 |
| Maximum Decrease | | |
| Before Treatment | 30% | 25% |
| After Treatment | 34% | 16% |
| Improvement | -15% | 37% |
| p-value | 0.05 | |
| Area Under Curve | | |
| Before Treatment | 1320 | 1319 |
| After Treatment | 1506 | 752 |
| Improvement | -14% | 43% |
| p-value | 0.02 | |

**Table II.**

| **LAR FEV₁ percent Change from Baseline in Allergen Challenge** | | |
|---|---|---|
| | Placebo | E25 |
| Total Number of Patients | 9 | 9 |
| Maximum Decrease | | |
| Before Treatment | 15% | 21% |
| After Treatment | 15% | 5% |
| Improvement | 0% | 76% |
| p-value | 0.05 | |
| Area Under Curve | | |
| Before Treatment | 2235 | 4928 |
| After Treatment | 2759 | 864 |
| Improvement | -23% | 82% |
| p-value | 0.04 | |

The effect of treatment with anti-IgE antibody was also apparent in the assessment of the concentration of allergen delivered during the second allergen challenge. These results, depicted in Table III, indicate that in 7/9 patients receiving E25 (as opposed to 2/9 patients receiving placebo), the dose of allergen necessary to achieve a 20% reduction in FEV₁ was increased by 100%.

**Table III.**

| **Concentration of Allergen Delivered During the Second Allergen Challenge** | | |
|---|---|---|
| | Placebo | E25 |
| Total Number of Patients | 9 | 9 |
| 100% Increase | 2 | 7 |
| Unchanged | 1 | 1 |
| Reduced by 50% | 5 | 0 |
| Reduced by 75% | 0 | 1 |
| Reduced by 88% | 1 | 0 |

In Canada, the primary efficacy endpoint was the EAR allergen PC₁₅ concentration (the allergen concentration needed to reach the 15% decrease in EAR FEV₁ after the allergen challenge). Basically, subjects inhaled increasing doubling doses at about 12 minute intervals until an FEV₁ measurement demonstrating a decrease of at least 15% or greater was obtained. The PC₁₅ was calculated using the last concentration of allergen (C2), the second last concentration of allergen (C1), the percent fall FEV₁ after C2 (R2) and the percent fall FEV₁ after C1 (R1) and the formula: log₁₀ allergen PC₁₅ = 0.3(15-R1)/(R2-R1) + log₁₀ C1. Baseline was defined as the allergen PC₁₅ concentration on Day-1. The primary efficacy variable was the change of allergen PC₁₅ concentration measured on Day 77. Between group differences for the change between Day-1 and Day 77 were assessed by the Wilcoxon Rank sum test. The changes of log allergen PC₁₅ measured within 1 hour of the allergen challenge on days 27 and 55 from baseline were also be compared between the two groups by the Wilcoxon Rank sum test.

Dosing for the subsequent challenges in the Canada protocol commenced at the same allergen challenge as the preceding challenge. However, if the FEV₁ did not fall by greater than or equal to 15% at the same concentration that caused a greater than or equal to 15 % fall during the first challenge, up to three additional doubling doses were delivered until a greater than or equal to 15% decrease was observed.

The results for the bronchial provocation testing with allergen in the Canada protocol are provided in tabular form in Table IV. The results demonstrate that patients receiving anti-IgE therapy required substantially more antigen (more doubling doses) to decrease their FEV₁ to at least 15% of baseline.

**Table IV.**

| **PC₁₅ Concentration Change** | | | |
|---|---|---|---|
| | | Placebo | E25 |
| Day 27 | < 1 doubling or | 75% | 10% |
| | no improvement | | |
| | > I doubling | 25% | 90% |
| | > 2 doublings | 0% | 60% |
| | > 3 doublings | 0% | 36% |
| | Median | <0 | 2.4 |
| | | | doublings |
| | p=0.0001 | | |
| Day 55 | < 1 doubling or | 89% | 20% |
| | no improvement | | |
| | > I doubling | 11% | 80% |
| | > 2 doublings | 0% | 60% |
| | > 3 doublings | 0% | 48% |
| | Median | 0 | 2.9 |
| | | | doublings |
| | p= 0.0008 | | |
| Day 77 | <1 doubling or | 67% | 20% |
| | no improvement | | |
| | > 1 doubling | 33% | 80% |
| | > 2 doublings | 11% | 70% |
| | > 3 doublings | 0% | 45% |
| | Median | <0 | 2.9 doublings |
| | p=0.002 | | |

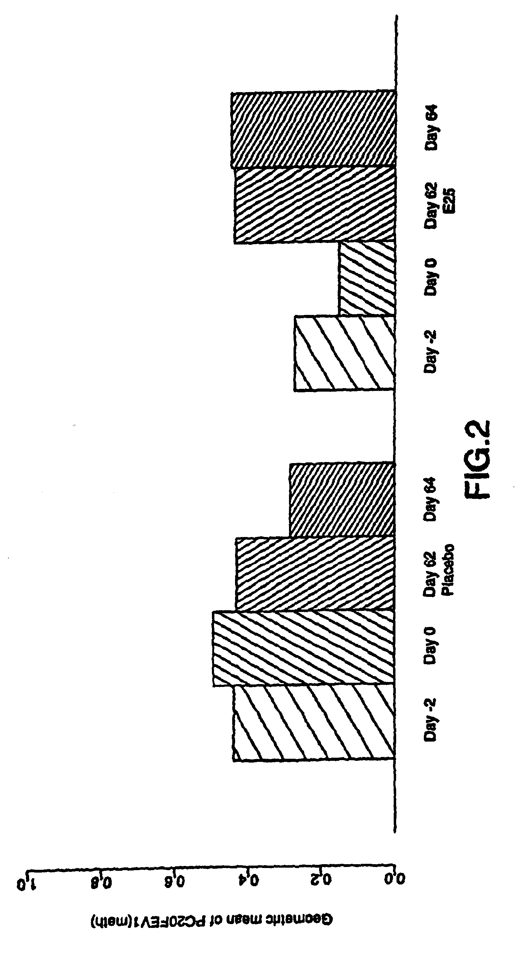

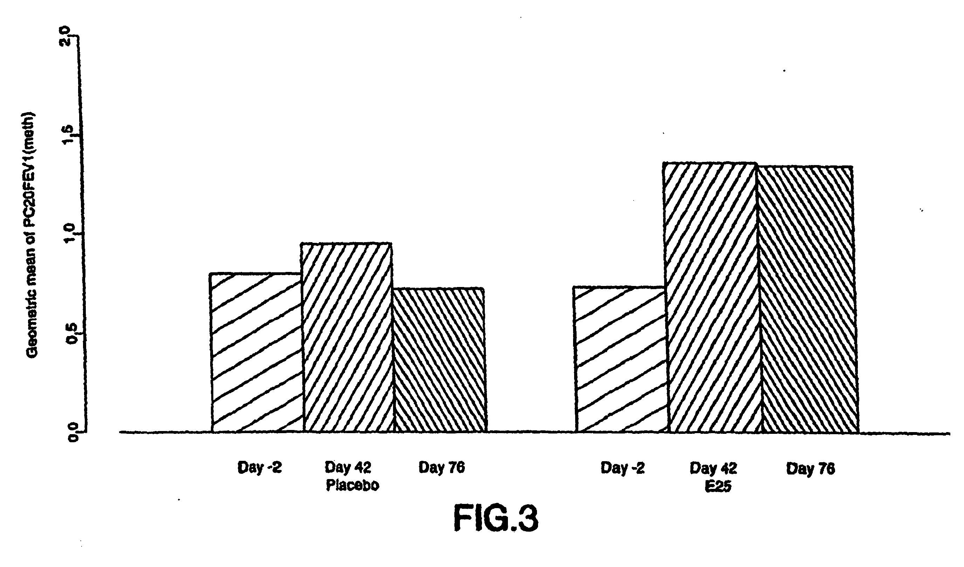

Figures 2 (U.S.) and 3 (Canada) depict the results from methacholine bronchial challenge in treated and untreated patients. Methacholine was delivered at an initial dose of 0.03 mg/ml and a dose-response curve was constructed by administering serial doubling concentrations of methacholine until the worst FEV₁ maneuver recorded 1 or 3 minutes after methacholine inhalation was less than or equal to 80% of the baseline FEV₁. The PC₂₀FEV₁ (methacholine) was calculated by linear interpolation between the last two points open the dose-response challenge. The results in Figures 2 and 3 indicate that as opposed to patients receiving placebo, a higher PC₂₀FEV₁(methacholine) was observed in patients receiving anti-IgE therapy. Thus, patients receiving anti-IgE antibody had reduced hyperresponsiveness as a result of therapy.

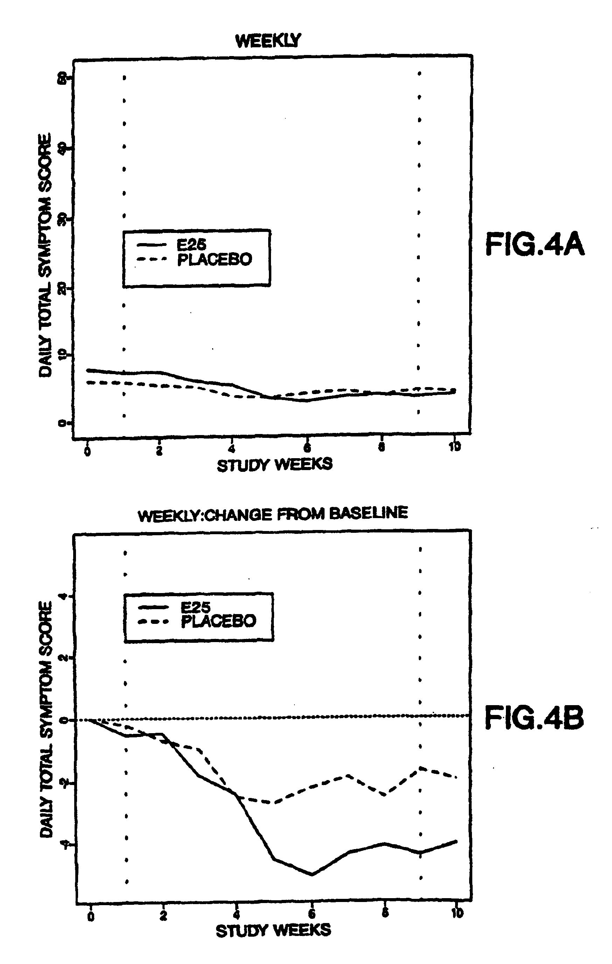

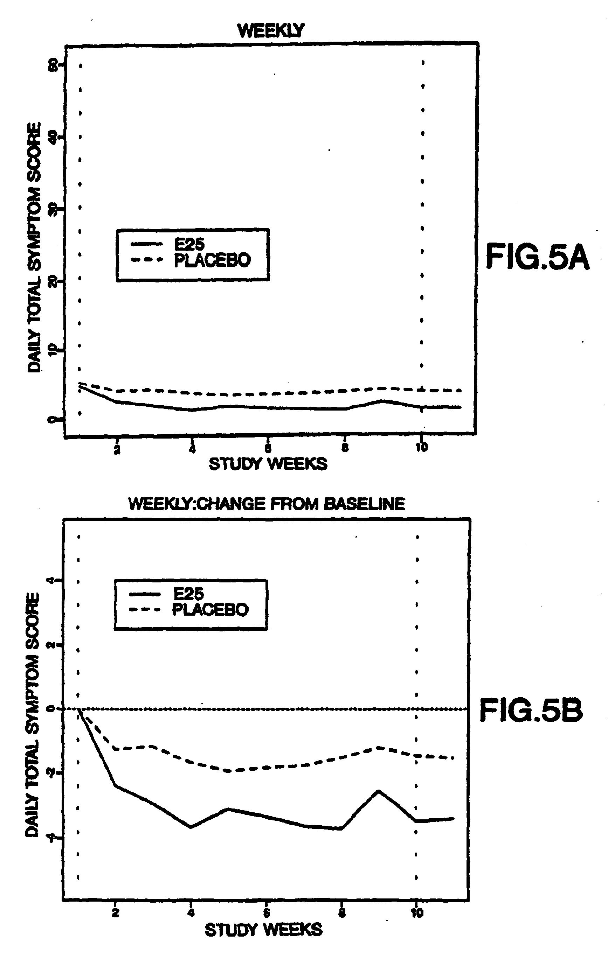

Figures 4 (U.S.) and 5 (Canada) depict the change from baseline in total symptoms scores. The subjects in the study were asked to maintain a daily symptoms diary. The parameters included symptoms of asthma such as shortness of breath, chest tightness, wheezing, cough, and sputum (phlegm/mucus): night-time asthma symptoms such as the number of time patient awoke with asthma. AM peak expiratory flow rate (best of three attempts), number of puffs of beta agonist inhaler in the past 12 hours; and day-time asthma symptoms such as absence from work due to asthma, PM peak expiratory flow rate (best of three attempts), and number of puffs of beta agonist inhaler in the past 12 hours. These symptoms were rated on a scale of 0-10, with 10 being extremely severe. The results in Figures 4 and 5 demonstrate that patients receiving anti-IgE therapy exhibit a trend of reduction in asthma related symptoms as a result of treatment

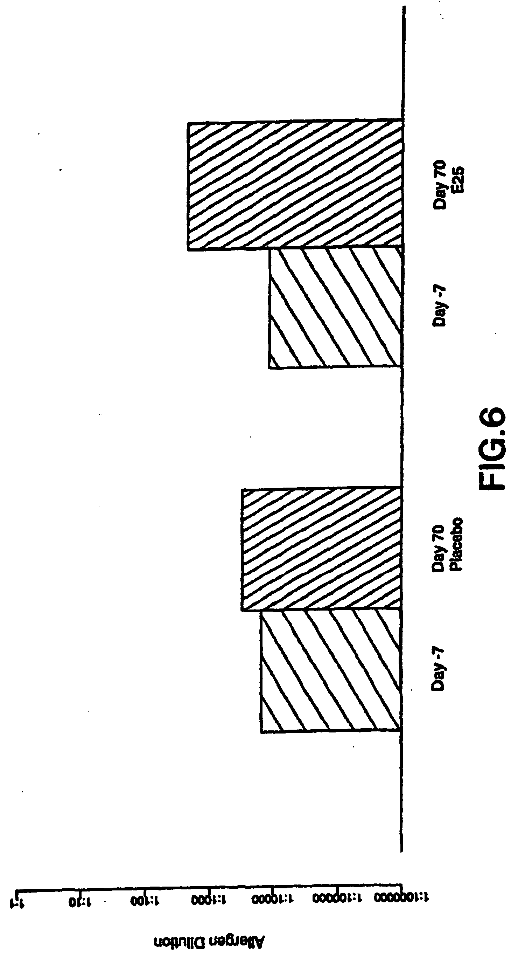

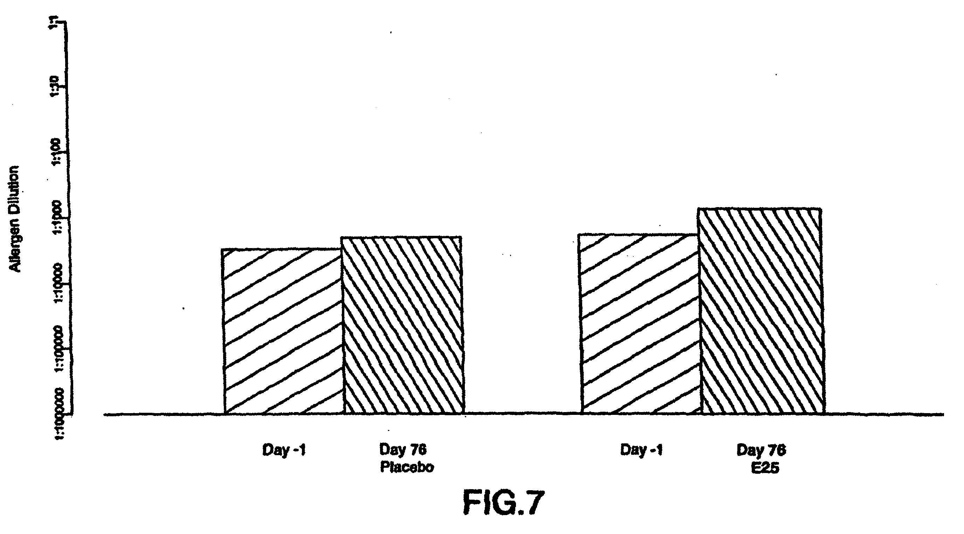

Figures 6 (U.S.) and 7 (Canada) depict endpoint titration skin testing for allergens in patients receiving placebo or anti-IgE antibody. Basically, patients were intradermally injected with serial tenfold dilutions of the allergen to which the patients were most reactive (house dust mite, cat hair, or grass) at Day -7 and Day 70 (after completion of treatment) to find the highest dilution that caused a skin reaction of 2 mm or less. The results demonsbate that subjects which received anti-IgE antibody had a substantially reduced reactivity to allergen as a result of treatment

In summary, the results from two dosing protocols demonstrated that treatment with anti-IgE significantly improved early and late asthmatic responses to allergen, non-specific bronchial hyperreactivity, and allergen skin test reactivity. Markers of airway inflammation were also improved.

## Claims

1. The use of an IgE antagonist in the preparation of a pharmaceutical composition for the reduction of the late asthmatic response of allergic asthma comprising a maintenance dose of an IgE antagonist, and optionally, a loading dose of the IgE antagonist, wherein the IgE antagonist acts by blocking the binding of IgE to its receptors on B cells, mast cells, or basophils by blocking the binding site on the IgE molecule; by binding soluble IgE ; or by binding to IgE on B cells and wherein the IgE antagonist is an anti-IgE antibody.

2. The use as claimed in claim 1 wherein the antibody is chimeric.

3. The use as claimed in claim 1 or claim 2 wherein the antibody is humanized or human.

4. The use as claimed in any one of the preceding claims, wherein the maintenance dose is repeated at intervals of about 1 to about 90 days.

5. The use as claimed in claim 4, wherein the maintenance dose is repeated weekly.

6. The use as claimed in claim 4, wherein the maintenance dose is repeated biweekly.

7. The use as claimed in any one of the preceding claims wherein the IgE antagonist binds to soluble IgE and blocks the binding of IgE to the IgE receptor on basophils.

8. The use as claimed in any one of the preceding claims, wherein the loading dose is administered before onset of asthma symptoms.

9. The use as claimed in any one of the preceding claims, wherein the loading dose is administered after the onset of asthma symptoms.

10. The use as claimed in any one of the preceding claims, wherein the loading dose, if present, is greater than the maintenance dose.

11. The use as claimed in any one of the preceding claims, wherein the antagonist is administered in a formulation comprising a buffer, a salt, optionally, a polyol, and optionally, a preservative.

12. The use as claimed in any one of the preceding claims, wherein the formulation is freeze-dried, then reconstituted before administration.

13. The use as claimed in any one of the preceding claims, wherein the maintenance dose, and the loading dose if present, reduce the concentration of free IgE in the patient's serum to less than about 40ng/ml.

14. The use as claimed in any one of the preceding claims wherein the maintenance dose of antagonist is about 0.001 to 0.01 mg/kg/week/baseline IgE IU/ml.

15. The use as claimed in any one of the preceding claims, wherein the maintenance dose, and the loading dose if present, results in a total serum concentration of antagonist of about 1 to 10 times greater than the patient's total serum IgE concentration.

16. The use as claimed in any one of the preceding claims, wherein the treatment comprises administering to the patient a dose of IgE antagonist averaging about 0.001 to 0.01 mg/kg/week IgE antagonist for every IU/ml baseline IgE in the patient's serum.

## Patentansprüche

1. Verwendung eines IgE-Antagonisten zur Herstellung einer pharmazeutischen Zusammensetzung zur Verringerung der späten asthmatischen Reaktion von allergischem Asthma, umfassend eine Erhaltungsdosis eines IgE-Antagonisten und gegebenenfalls eine Ladungsdosis des IgE-Antagonisten, worin der IgE-Antagonist wirkt, indem er die Bindung von IgE an seine Rezeptoren auf B-Zellen, Mastzellen oder Basophilen durch Blockieren der Bindungsstelle auf dem IgE-Molekül blockiert; indem er lösliches IgE bindet; oder indem er an IgE auf B-Zellen bindet, und worin der IgE-Antagonist ein Anti-IgE-Antikörper ist.

2. Verwendung nach Anspruch 1, worin der Antikörper chimär ist.

3. Verwendung nach Anspruch 1 oder 2, worin der Antikörper humanisiert oder human ist.

4. Verwendung nach einem der vorangegangenen Ansprüche, worin die Erhaltungsdosis in Intervallen von etwa 1 Tag bis etwa 90 Tagen wiederholt wird.

5. Verwendung nach Anspruch 4, worin die Erhaltungsdosis wöchentlich wiederholt wird.

6. Verwendung nach Anspruch 4, worin die Erhaltungsdosis alle zwei Wochen wiederholt wird.

7. Verwendung nach einem der vorangegangenen Ansprüche, worin sich der IgE-Antagonist an lösliches IgE bindet und die Bindung von IgE an den IgE-Rezeptor auf Basophilen blockiert.

8. Verwendung nach einem der vorangegangenen Ansprüche, worin die Ladungsdosis vor dem Einsetzen von Asthmasymptomen verabreicht wird.

9. Verwendung nach einem der vorangegangenen Ansprüche, worin die Ladungsdosis nach dem Einsetzen von Asthmasymptomen verabreicht wird.

10. Verwendung nach einem der vorangegangenen Ansprüche, worin die Ladungsdosis, sofern vorhanden, stärker als die Erhaltungsdosis ist.

11. Verwendung nach einem der vorangegangenen Ansprüche, worin der Antagonist in einer Formulierung verabreicht wird, die einen Puffer, ein Salz, gegebenenfalls ein Polyol und gegebenenfalls ein Konservierungsmittel umfasst.

12. Verwendung nach einem der vorangegangenen Ansprüche, worin die Formulierung gefriergetrocknet und dann vor der Verabreichung rekonstituiert wird.

13. Verwendung nach einem der vorangegangenen Ansprüche, worin die Erhaltungsdosis und die Ladungsdosis, sofern vorhanden, die Konzentration von freiem IgE im Serum des Patienten auf weniger als etwa 40 ng/ml reduzieren.

14. Verwendung nach einem der vorangegangenen Ansprüche, worin die Erhaltungsdosis an Antagonist etwa 0,001 bis 0,01 mg/kg/Woche/Grundlinien-IgE-IE/ml beträgt.

15. Verwendung nach einem der vorangegangenen Ansprüche, worin die Erhaltungsdosis und die Ladungsdosis, sofern vorhanden, eine Antagonist-Gesamtkonzentration im Serum ergeben, die etwa 1- bis 10-mal höher als die IgE-Gesamtkonzentration im Serum des Patienten ist.

16. Verwendung nach einem der vorangegangenen Ansprüche, worin die Behandlung die Verabreichung einer Dosis an IgE-Antagonisten an den Patienten umfasst, die durchschnittlich etwa 0,001 bis 0,01 mg/kg/Woche an IgE-Antagonist pro IE/ml Grundlinien-IgE im Serum des Patienten beträgt.

## Revendications

1. Utilisation d'un antagoniste de IgE dans la préparation d'une composition pharmaceutique destinée à la réduction de la réponse asthmatique tardive de l'asthme allergique, comprenant une dose d'entretien d'un antagoniste de IgE et, facultativement, une dose d'attaque de l'antagoniste de IgE, dans laquelle l'antagoniste de IgE agit en bloquant la liaison de la IgE à ses récepteurs sur les lymphocytes B, les mastocytes ou les cellules basophiles, en bloquant le site de liaison sur la molécule de IgE, par liaison à la IgE soluble ; ou par liaison à la IgE sur les lymphocytes B, et dans laquelle l'antagoniste de IgE est un anticorps anti-IgE.

2. Utilisation suivant la revendication 1, dans laquelle l'anticorps est un anticorps chimère.

3. Utilisation suivant la revendication 1 ou la revendication 2, dans laquelle l'anticorps est un anticorps humanisé ou humain.

4. Utilisation suivant l'une quelconque des revendications précédentes, dans laquelle la dose d'entretien est répétée à des intervalles d'environ 1 à environ 90 jours.

5. Utilisation suivant la revendication 4, dans laquelle la dose d'entretien est répétée de manière hebdomadaire.

6. Utilisation suivant la revendication 4, dans laquelle la dose d'entretien est répétée tous les 15 jours.

7. Utilisation suivant l'une quelconque des revendications précédentes, dans laquelle l'antagoniste de IgE se lie à une IgE soluble et bloque la liaison de la IgE au récepteur de IgE sur les cellules basophiles.

8. Utilisation suivant l'une quelconque des revendications précédentes, dans laquelle la dose d'attaque est administrée avant le déclenchement des symptômes de l'asthme.

9. Utilisation suivant l'une quelconque des revendications précédentes, dans laquelle la dose d'attaque est administrée après le déclenchement des symptômes de l'asthme.

10. Utilisation suivant l'une quelconque des revendications précédentes, dans laquelle la dose d'attaque, si elle est présente, est supérieure à la dose d'entretien.

11. Utilisation suivant l'une quelconque des revendications précédentes, dans laquelle l'antagoniste est administré dans une formulation comprenant un tampon, un sel, facultativement un polyol et facultativement un conservateur.

12. Utilisation suivant l'une quelconque des revendications précédentes, dans laquelle la formulation est lyophilisée, puis est reconstituée avant administration.

13. Utilisation suivant l'une quelconque des revendications précédentes, dans laquelle la dose d'entretien, et la dose d'attaque si elle est présente, réduisent la concentration de IgE libre dans le sérum du patient à une valeur inférieure à environ 40 ng/ml.

14. Utilisation suivant l'une quelconque des revendications précédentes, dans laquelle la dose d'entretien de l'antagoniste est comprise dans l'intervalle d'environ 0,001 à 0,01 mg/kg/semaine/UI de Ige de base/ml.

15. Utilisation suivant l'une quelconque des revendications précédentes, dans laquelle la dose d'entretien, et la dose d'attaque si elle est présente, ont pour résultat une concentration sérique totale de l'antagoniste d'environ 1 à 10 fois supérieure à la concentration sérique totale de IgE du patient.

16. Utilisation suivant l'une quelconque des revendications précédentes, dans laquelle le traitement comprend l'administration au patient d'une dose d'antagoniste de IgE en moyenne d'environ 0,001 à 0,01 mg/kg/semaine d'antagoniste de IgE pour chaque IU/ml de IgE de base dans le sérum du patient.
